(19)

**Europäisches Patentamt**
**European Patent Office**
**Office européen des brevets**

(11) **EP 4 459 494 B1**

(12) **EUROPEAN PATENT SPECIFICATION**

(45) Date of publication and mention
of the grant of the patent:
**28.01.2026 Bulletin 2026/05**

(21) Application number: **24174154.5**

(22) Date of filing: **03.05.2024**

(51) International Patent Classification (IPC):
**G06F 30/10** (2020.01)   **G06F 30/25** (2020.01)
**G16C 60/00** (2019.01)   **G01N 23/046** (2018.01)

(52) Cooperative Patent Classification (CPC):
**G06F 30/25; G06F 30/10; G16C 60/00;**
G01N 23/046

(54) **SYSTEM AND METHOD FOR GENERATING A VIRTUAL TWIN OF A POROUS MATERIAL IMAGE FILE**

SYSTEM UND VERFAHREN ZUR ERZEUGUNG EINES VIRTUELLEN ZWILLINGS EINER BILDDATEI EINES PORÖSEN MATERIALS

SYSTÈME ET PROCÉDÉ DE GÉNÉRATION D'UN JUMEAU VIRTUEL D'UN FICHIER D'IMAGE DE MATÉRIAU POREUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.05.2023 US 202318312388**

(43) Date of publication of application:
**06.11.2024 Bulletin 2024/45**

(73) Proprietor: **Dassault Systèmes Americas Corp.**
**Waltham, MA 02451 (US)**

(72) Inventors:
• **Han, Jaebeom**
**Ludlow (US)**
• **Subramanian, Lalitha**
**Newtown (US)**
• **Todd, Stephen**
**Cambridgeshire (GB)**
• **Salazar-Tio, Rafael**
**San Ramon (US)**
• **Balasubramanian, Ganapathi Raman**
**Pleasanton (US)**
• **Skinner, Kwan**
**Mesa (US)**
• **Schweizer, Sabine**
**Angelbachtal (DE)**

(74) Representative: **Bandpay & Greuter**
**11 rue Christophe Colomb**
**75008 Paris (FR)**

(56) References cited:
**CN-B- 102 087 676     US-A1- 2011 004 447**

• **MOON JOSEPH B ET AL: "A fast algorithm for generating smooth molecular dot surface representations", vol. 7, 1 June 1989 (1989-06-01), pages 109 - 112, XP093207363, Retrieved from the Internet <URL:https://pdf. sciencedirectassets.com/271978/ 1-s2.0-S0263785589X80018/ 1-s2.0-S0263785589800141/main.pdf? X-Amz-Security-Token=IQoJb3JpZ2luX2VjEC QaCXVzLWVhc3QtMSJGMEQCICT+UUo21Uvx9 MglHz2ii8mODSOtr2HWrLACgedzfcutAiBHqPld dYKfA1w98vXxgouikAKI/+iyJweTzaXpd54 WYiqzBQhdEAUaDDA1OTAwMzU0Njg2NSIM5A2 Xn/o7bSKE/6L7K> [retrieved on 20240920]**

• **TIAGO SIM◇ES ET AL: "Geometric Detection Algorithms for Cavities on Protein Surfaces in Molecular Graphics: A Survey", COMPUTER GRAPHICS FORUM : JOURNAL OF THE EUROPEAN ASSOCIATION FOR COMPUTER GRAPHICS, WILEY-BLACKWELL, OXFORD, vol. 36, no. 8, 1 June 2017 (2017-06-01), pages 643 - 683, XP071489622, ISSN: 0167-7055, DOI: 10.1111/CGF.13158**

• **ALMETWALLY A G ET AL: "Experimental investigation of 3D printed rock samples replicas", JOURNAL OF NATURAL GAS SCIENCE AND ENGINEERING, ELSEVIER, AMSTERDAM, NL, vol. 76, 5 February 2020 (2020-02-05), XP086084481, ISSN: 1875-5100, [retrieved on 20200205], DOI: 10.1016/ J.JNGSE.2020.103192**

**Description**

FIELD OF THE INVENTION

The present invention relates to chemistry based 3D modeling of porous materials, and more particularly, is related to imaging of a modeled microstructure.

BACKGROUND OF THE INVENTION

The original and typical workflow in analyzing porous materials begins with the use of experimental microscopy 3D imaging techniques, such as micro-CT (micrometer computed tomography), and FIB-SEM (focus ion-beam scanning electron microscopy). These experimental 3D images are utilized to reconstruct the 3D structure of the material to be analyzed. However, this image based workflow is not applicable for any material where experimental data are not available, such as newly designed materials, non-existent and virtually designed materials, and porous materials with nanopores below the limits of typical experimental measurements. While the lattice Boltzman method (LBM) based application has been used for the fluid flow simulation for battery electrode structures that were generated from coarse-grained simulation methods, the LBM method does not include a general framework.

Experimental micro-CT images or FIB-SEM images are often unavailable, or are only measured at ambient conditions and not in-situ conditions where the temperature and pressure conditions may be much higher than ambient conditions. This can affect the actual microstructure to be imaged. In addition, such experiments have a limitation on the size of the pores detected (for example, on the order of 0.01 micrometers), and many times these smaller pores coexist with larger pores orders of magnitude larger that also need to be statistically represented in the captured images. Further, it is difficult and expensive to scan and produce images of pores in the nanometer scales. Moreover, depending on the inherent chemistry of the material, the pore sizes, shapes, distribution, channels, etc., may vary under different conditions. Therefore, there is a need in the industry to synthesize accurate CT-like images for newly designed materials, non-existent and virtually designed materials, and/or porous materials with nanopores below the limits of typical experimental measurements. TIAGO SIMÕES ET AL: "Geometric Detection Algorithms for Cavities on Protein Surfaces in Molecular Graphics: A Survey" discloses molecular modelling and analysis.

SUMMARY OF THE INVENTION

The invention is defined by the independent claims.

Embodiments of the present invention provide a system and method for generating virtual twins of porous materials. Briefly described, the present invention is directed to producing a pseudo micro computed tomography (CT-like) image of a porous material. A chemistry-based 3D structure of a porous material system is generated, and a Connolly surface for the 3D structure is determined. A volume field of the 3D chemistry-based structure is calculated from the Connolly surface. A text-format file layer having layer by layer information of the volume field is generated. The text-format layer file is converted into a CT-like binary image file in the RAW format. The binary image file is converted to a black and white or grayscale images. A pore size analysis (PSA) simulation is performed to produce grain images and pore images for the porous material system.

A system implementing the present invention facilitates investigation of structural properties of porous materials and flow properties in porous materials without any CT-like images from experiments.

In example, it is provided a system for modeling a porous material, generating a pseudo micro computed tomography (CT-like) image of the porous material, and analyzing the porous material for manufacture, comprising a processor and a memory configured to store non-transitory instructions that, when executed by the processor, cause the system to perform the method for generating virtual twins of porous materials. In another example, it is provided a system for modeling a porous material, comprising a processor and a memory configured to store non-transitory instructions that, when executed by the processor, cause the system to perform the method for generating virtual twins of porous materials.

BRIEF DESCRIPTION OF THE DRAWINGS

The accompanying drawings are included to provide a further understanding of the invention, and are incorporated in and constitute a part of this specification. The components in the drawings are not necessarily to scale, emphasis instead being placed upon clearly illustrating the principles of the present invention. The drawings illustrate embodiments of the invention and, together with the description, serve to explain the principles of the invention.

FIG. 1A is a schematic diagram showing a 3D structure of a porous electrode generated from Mesocite in Materials Studio.

FIG. 1B is a schematic diagram showing a 3D structure of a porous electrode generated from LAMMPS.

FIG. 2A is a schematic diagram showing generation of volume field data from the 3D structure of FIG. 1A.

FIG. 2B is a schematic diagram showing generation of volume field data from the 3D structure of FIG. 1B.

FIG. 3A is a schematic diagram showing generation of an image file from the volume field data from the volume field data of FIG. 2A.

FIG. 3B is a schematic diagram showing generation of an image file from the volume field data from the volume field data of FIG. 2B.

FIG. 4A shows side-by-side original and processed images of the 3D structure from Materials Studio (Mesocite module) in DigitalROCK.

FIG. 4B shows side-by-side original and processed images of the 3D structure from LAMMPS (DPP2 model) in DigitalROCK.

FIG. 5A shows diagrams depicting grain (top), pore sites (center), and pore size distribution plot (bottom) of the 3D structure from Mesocite in Materials Studio.

FIG. 5B shows diagrams depicting grain (top), pore sites (center), and pore size distribution plot (bottom) of the 3D structure from the DPP2 model in LAMMPS.

FIG. 5C shows graphical pore size distribution of the 3D structure from Mesocite as per FIG. 5A.

FIG. 5D shows graphical pore size distribution of the 3D structure from the DPP2 model as per FIG. 5B.

FIG. 6 shows a visualized pore structure of the material saturated by a liquid at a specific saturated concentration (top left), a plot of the liquid saturation concentration versus location in height, (top right) and the capillary pressure plot with respect to the liquid saturation (bottom).

FIG. 7 is a flowchart of an exemplary method embodiment for generating a CT-like image for a porous material.

FIG. 8 is a flowchart of an exemplary method for converting the volume field to a text-format file as per FIG. 7.

FIG. 9 is a flowchart of an exemplary method for converting the text-format layer file to binary image file to a text-format file, as per FIG. 7.

FIG. 10 is a schematic diagram illustrating an example of a system for executing functionality of the present invention.

FIG. 11 is a schematic diagram of an exemplary embodiment of a system for modeling a porous material, generating a pseudo micro computed tomography (CT-like) image of the porous material, and analyzing the porous material for manufacture.

## DETAILED DESCRIPTION

The following definitions are useful for interpreting terms applied to features of the embodiments disclosed herein, and are meant only to define elements within the disclosure.

As used within this disclosure, Micro-CT refers to micrometer computer tomography, FIB-SEM refers to focus ion-beam scanning electron microscopy, and LBM refers to the Lattice Boltzman method (lattice based application fluid flow simulation). The images produced by micro-CT are referred to herein as CT images. In general, a CT scan uses X-rays and computers to produce a CT image made up of a series of layered images of a cross-section of the subject. Previously, it has been necessary to use samples of real-world porous materials for Micro-CT or FIB-SEM to produce FIB-SEM images and CT-images.

As used within this disclosure, a "CT-like image" or a "pseudo micro computed tomography image" refers to image data of a porous material in the format of a CT image obtained via modeling the porous material, rather than performing actual CT or FIB-SEM imaging operations on a sample of a porous material. A CT-like image may be used in place of a CT image for subsequent processing and/or simulation.

Three types of simulations are referred to in this disclosure: atomistic, coarse-grained, and dissipative particle dynamics (DPD).

Regarding atomistic simulations, the Atomic Simulation Environment (ASE) is a set of tools and modules for setting up, manipulating, running, visualizing, and analyzing atomistic simulations. The code is freely available under an open source license. ASE provides interfaces to different codes through calculators which are used together with the central Atoms object and the many available algorithms in ASE.

Coarse-grained modeling/models aim at simulating the behavior of complex systems using a coarse-grained (simplified) representation.

Regarding DPD emulations, dissipative particle dynamics (DPD) is an off-lattice mesoscopic simulation technique involving a set of particles moving in continuous space and discrete time. Particles represent whole molecules or fluid regions, rather than single atoms, and atomistic details are not considered relevant to the processes addressed. The internal degrees of freedom of the particles are integrated out and replaced by simplified pairwise dissipative and random forces to conserve momentum locally and ensure correct hydrodynamic behavior. This method advantageously gives access to longer time and length scales than are possible using conventional molecular dynamics (MD) simulations. Simulations of polymeric fluids in volumes up to 100 nm in linear dimension for tens of microseconds are possible.

As used within this disclosure, "Dynamic particle packing 2 (DPP2)" refers to a model was developed to predict the microstructure of Li-ion batteries, in particular, the cathode component of the batteries, and may be used to optimize the capacity, cycle life, and safety of such batteries. DPP2 improves upon the DPP1 dynamic particle packing (DPP) microstructure model. The DPP1 model simulates the final or dried electrode structure by moving spherical particles under periodic boundaries using Newton's laws of motion. DPP2 also includes solvent effects and is used to simulate the slurry-coating, drying, and calendaring processes.

As used within this disclosure, "LAMMPS (Large-scale Atomic/Molecular Massively Parallel Simulator)" refers to a classical molecular dynamics code with a focus on materials modeling.

As used within this disclosure, "mesoscale" refers to molecules/pores having a diameter range between 10 nm and 100 $\mu$m.

As used within this disclosure, a "Connolly surface" refers to a surface defining a boundary between a molecular structure and its environment. The shape of the Connolly surface depends on the radius of the probe used to inspect the structure.

As used within this disclosure, "BIOVIA" refers to a United States based software company that provides software for chemical, materials, and bioscience research for the pharmaceutical, biotechnology, consumer packaged goods, aerospace, energy, and chemical industries.

As used within this disclosure, "Materials Studio" refers to a commercial software based platform for simulating and modeling materials. Materials Studio is developed and distributed by BIOVIA.

As used within this disclosure, "Forcite" is a classical molecular mechanics tool with BIOVIA Materials Studio that calculates geometry optimization and dynamical, thermodynamical, and mechanical properties of molecules and periodic systems. For example, Forcite has modules to perform energy minimization and equilibration on a modeled material.

As used within this disclosure, a "canonical condition" refers to coarse grained models subjected to conditions (such as temperature, pressure, etc.) similar to those used for atomistic simulations. As used within this disclosure, a "RAW file" refers to an image file where the image data is uncompressed and unprocessed image, for example, data captured by a digital camera or a scanner sensor.

Reference will now be made in detail to embodiments of the present invention, examples of which are illustrated in the accompanying drawings. Wherever possible, the same reference numbers are used in the drawings and the description to refer to the same or like parts.

Embodiments of the present invention produced CT-like images synthesized from porous material models based on the inherent chemistry of the material at micrometer scale lengths. The embodiments enable modeling and optimization of flow properties of porous material at microscale level to produce CT-like images without experimental information. Previously, images of material samples were needed to model flow behavior. The images were obtained from, for example, micro-CT and/or FIB/SEM. Exemplary embodiments of the present invention use models based on 3D chemistry as input in lieu of images of physical material samples. The embodiments may incorporate a fully automated optimization process, for example, by performing all steps in a data modeling and simulation ecosystem such as Dassault's 3DEXPERIENCE platform.

The embodiments enable researchers to investigate the structural properties of porous materials and flow properties in porous materials without performing any experiments on real-life porous materials. This provides the capability for screening microporous materials that have not been synthesized (or purchased), and to determine the behavior of microporous materials under extreme conditions of temperature and pressure which are difficult or impossible to measure experimentally. The exemplary embodiments described below are directed to materials represented as spherical particles and/or different sizes of spherical particles. Non-spherical particles may be modeled in alternative embodiments.

This embodiments incorporate a workflow where chemistry based virtual 3D models of the porous materials are simulated and converted to CT-like images, for example, using BIOVIA's Mesocite module in Materials Studio and/or LAMMPS and/or GROMACS and/or other discrete element method (DEM) packages and/or other coarse-grain packages. The images are received as input by software for modeling flow properties of porous materials based on image analysis, for example, SIMULIA's DigitalROCK or other modeling software. Moreover, this workflow can be automated within a simulation optimization ecosystem such as the 3DEXPERIENCE platform for the purpose of global optimization, including the materials and microstructure.

The embodiments provide a "model-first" approach that reduces the need to perform time-consuming, wasteful, and expensive experiments. Furthermore, when the embodiments are implemented in the cloud, for example, by implementing the workflow in 3DEXPERIENCE, the chemistry-based porous media virtual twin experiences may accelerate innovation for a number of industries.

Modeling of model porous materials have been built on the foundation of experimental results. However, the experiments producing these results are often limited by several factors, including difficulties obtaining experimental results that correctly reflect real-world conditions and scenarios. Furthermore, experiments may be difficult or impractical to conduct, or the experiments may produce unreliable results for very small particle and pore sizes, for example, at or below micrometer scale. The exemplary embodiments more accurately model porous materials based on the inherent chemistry and to model the porous material down to and below micrometer scale lengths.

The exemplary method embodiments presented below are described in the context of a GUI-based CAD system, for example, the Dassault Systemes 3DEXPERIENCE platform in the cloud computing environment for steps 4 and 5 (see below). It is also possible to perform these steps via a script in command-line interface, if desired.

Under a first step ("Step 1"), a chemistry-based 3D structure of a porous material system is generated using computational chemistry methods. The first exemplary embodiment is directed to modeling structures at mesoscale level, but there is no restriction on scales and on computational methods for alternative embodiments. Under the first embodiments, two mesoscale porous electrode structures may be employed in this description from two different simulation methods, for example 1) the dissipative particle dynamics (DPD) simulation using the Mesocite module in Materials Studio, and 2) the coarse-grained (CG) simulation based on the dynamic particle packing 2 (DPP2) model using LAMMPS.

For example, using Mesocite in Dassault Systemes BIOVIA Materials Studio, first construct a mesoscale 3D structure using the Mesostructure tools in Materials Studio. Then create a DPD forcefield (input parameters) using the solubility parameter of each particle in the system. For example, the solubility parameter may be obtained from experimental data or may be calculated either from atomistic molecular dynamics (MD) simulations using the Forcite module in Materials Studio or from COSMOtherm. Solubility parameters of polymers may also be obtained from Synthia in Materials Studio. DPD simulations are performed after energy minimization and equilibration, and obtain mechanical properties using Materials Studio.

In cases where experimental mechanical properties of similar materials is available, the user may make a direct comparison of the simulated and experimental results.

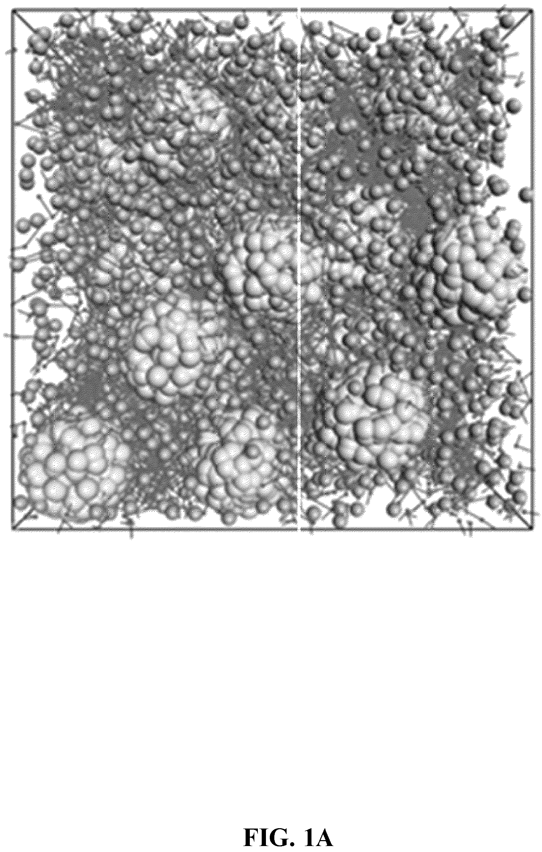

For any systems that experimental data are not available, the properties obtained from all-atom molecular dynamics (MD) simulations of each compound should be utilized as reference. FIG.1A shows a 3D Structure of a porous electrode generated from Mesocite in Materials Studio.

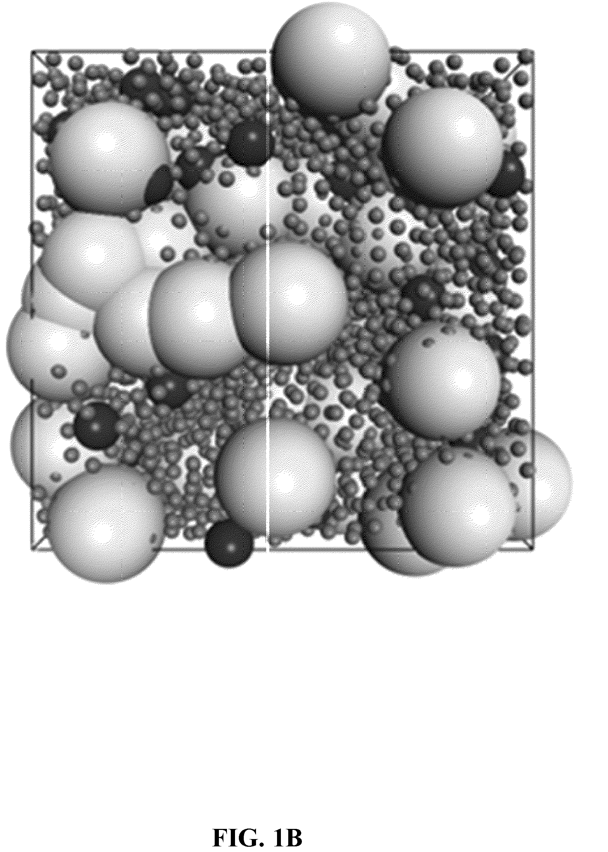

A coarse-grained DPP2 model may be created in LAMMPS as follows. First, a coarse-grained initial structure is constructed by placing particles randomly inside the box, where the box refers to a three dimensional representative unit in x-, y-, z- dimensions. Because of the computational cost, it is not practical to model a whole chemical, materials, or biological systems, while it is practical (and reasonable) to set up a system with a small portion of the molecular system of interest, under the assumption the real system can be approximated to a periodically replicated system from the modeled structure in a box. The box size is first set to a little larger size than the size calculated from a target density because the box size may be adjusted during the simulation to match the density of the model system to the real or target system. A CG MD simulation may then be run in the isothermal isobaric condition after energy minimization (for example, using Forcite) in order to adjust the density of the material. In cases where experimental density is available, a direct comparison of the simulated and experimental results can be made. If required, the user may adjust DPP2 parameters to obtain a better match between simulated and experimental results. For systems where experimental data are not available, the properties obtained from all-atom molecular dynamics (MD) simulations of each compound may be utilized as reference. A CG simulation is then run using the equilibrated structure above in the canonical condition, and structural and mechanical properties are obtained for the user to validate the model. FIG. 1B shows a 3D Structure of a porous electrode generated from LAMMPS.

Under a second step of the first exemplary embodiment ("step 2"), the volume field of a 3D structure is calculated based on the Connolly surface of the modeled structure, as is familiar to persons having skill in the art. The volume field is used as a metric to decide if a certain position is occupied by a material or a particle, of a target molecular structure.

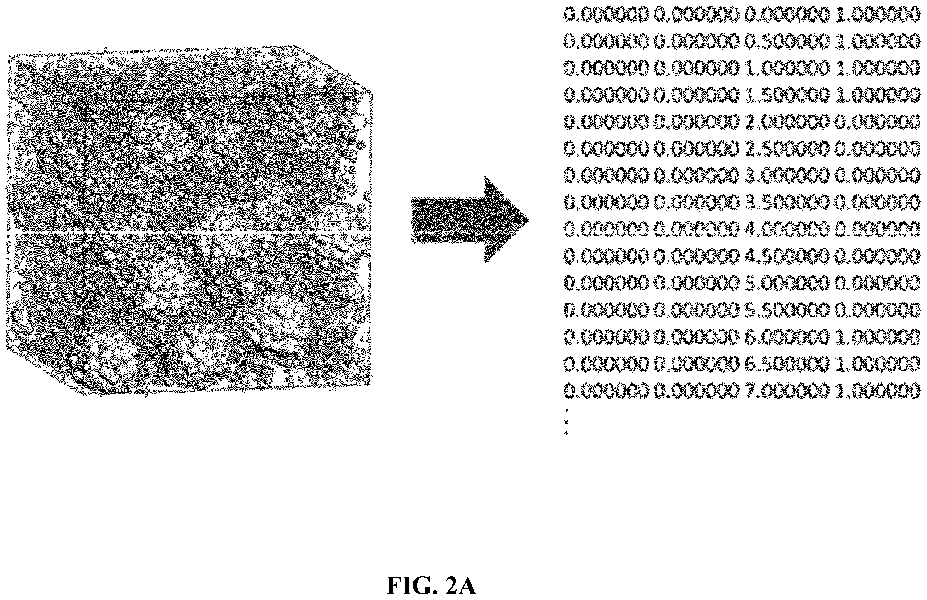

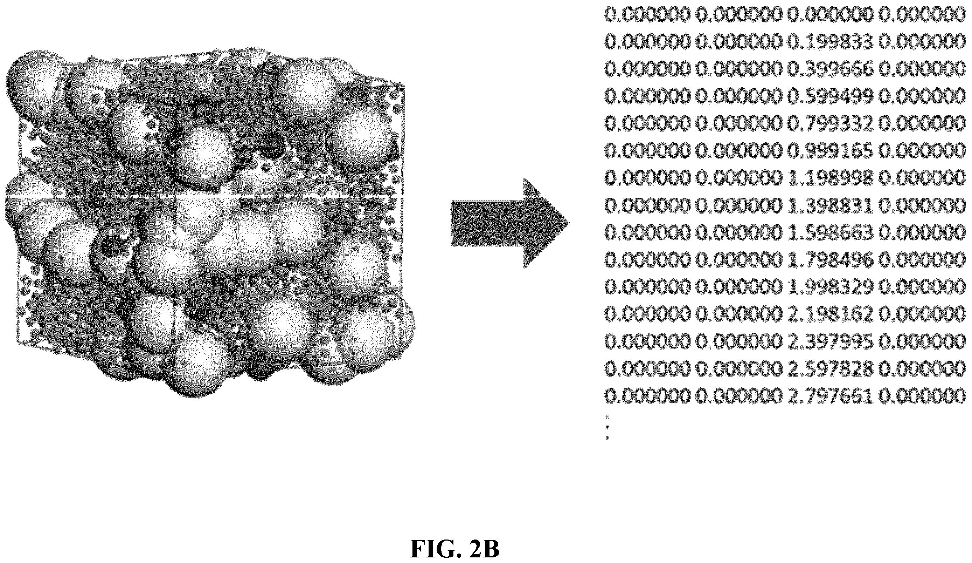

A text-format file is generated containing the 3D structure volume field information layer by layer, for example, by using a script in BIOVIA software. FIG. 2A depicts generation of volume field data from the 3D structure simulated using Mesocite in Materials Studio. In the text-format file, the first three columns represent the coordinate of the 3D structure or the pixel location in the image file, and the last column shows if this position is occupied by a particle (0) or a pore (1). FIG. 2B depicts generation of volume field data from the 3D structure simulated using the DPP2 model in LAMMPS.

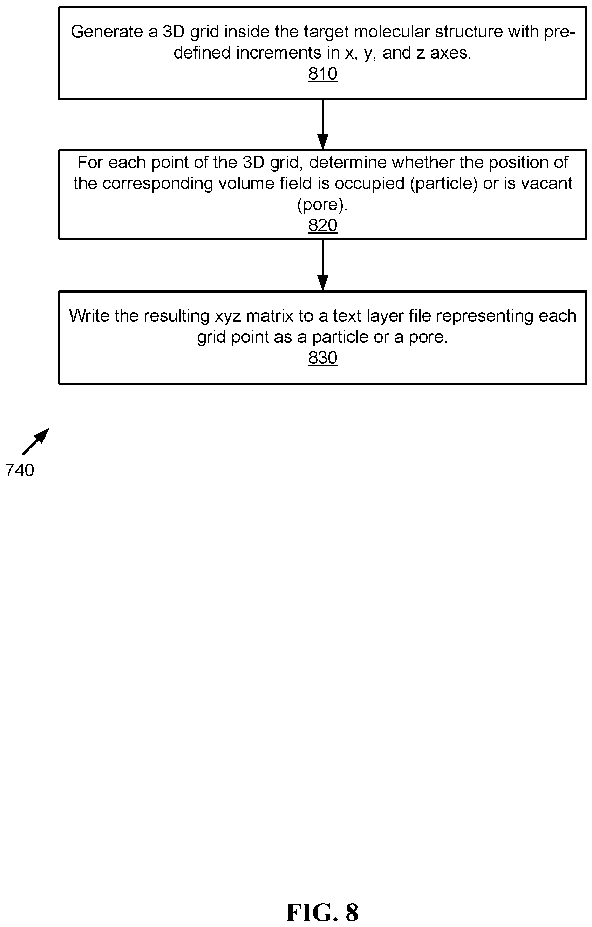

FIG. 8 is a flowchart of an exemplary method embodiment for converting the volume field to a text-format file. Here, the volume field is sliced into layers (z-axis), and then sectioned into a grid in an x-y plane. It should be noted that any process descriptions or blocks in flowcharts should be understood as representing modules, segments, portions of code, or steps that include one or more instructions for implementing specific logical functions in the process, as would be understood by those reasonably skilled in the art of the present invention.

Points for a 3D grid are generated inside the target molecular structure with pre-defined increments in x, y, and z axes, as shown by block 810. For each point of the 3D grid, determine if the corresponding volume field value is occupied by a particle (volume field <= 0.0) or is vacant, or a pore, (volume field > 0.0), as shown by block 820. The resulting xyz matrix is printed to text-format layer a file in a binary format (0: particle, 1: pore) at each position, as shown by block 830.

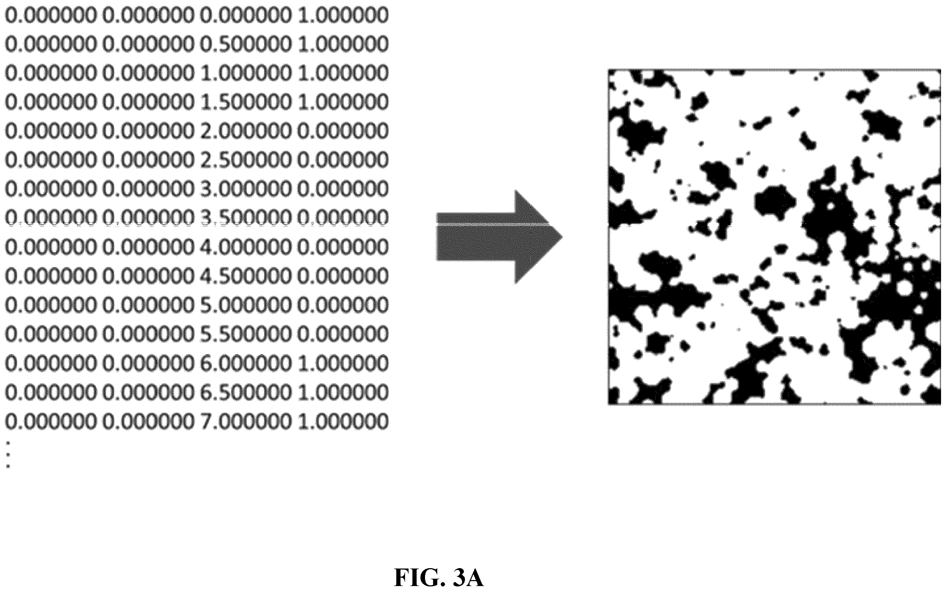

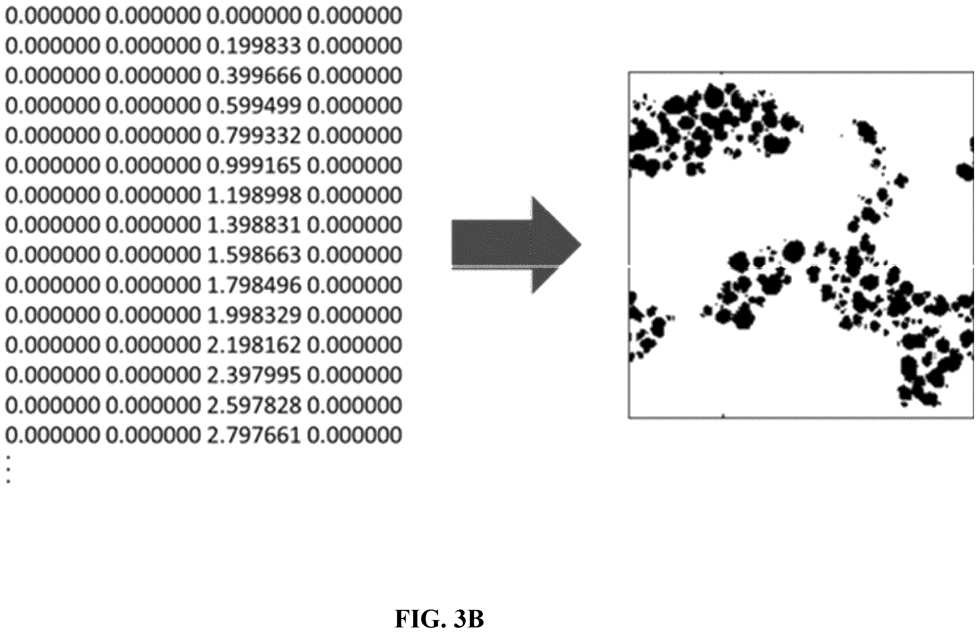

Under a third step of the first exemplary embodiment ("step 3"), the text-format layer file is converted to a binary image file in the RAW image format, as described further below. In the image file shown by FIGS. 3A and 3B, the white area represents the particles, and the black area represents the pores.

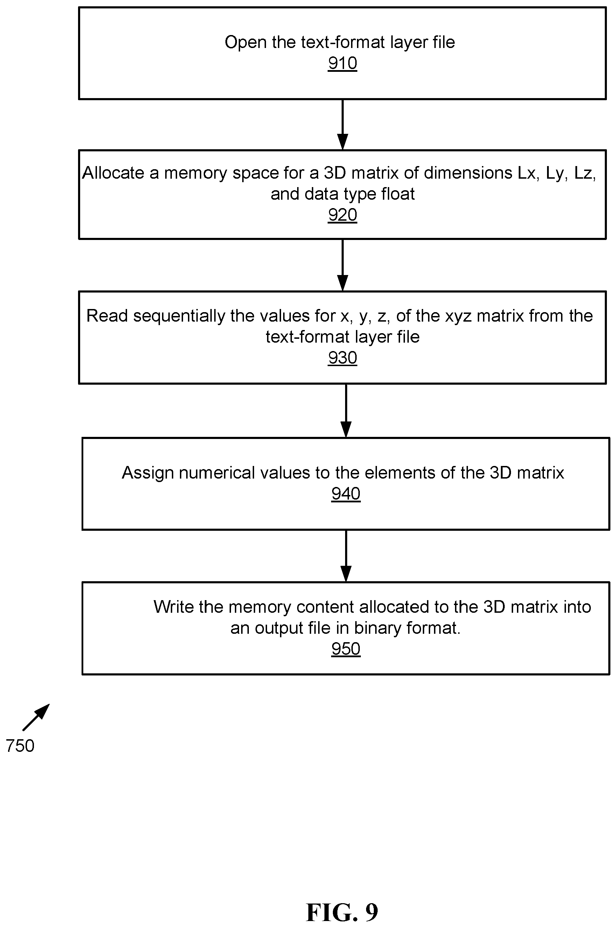

FIG. 9 is a flowchart of an exemplary method for converting the text-format layer file to binary image file to a text-format file. The text-format layer file from block 830 (FIG. 8) is opened, as shown by block 910. A memory space is allocated for a 3D matrix of dimensions Lx, Ly, Lz, and data type float, as shown by block 920. The values for x, y, z, are sequentially read from the xyz matrix _xyz of the text-format layer file, as shown by block 930. Numerical values are assigned to the elements of each layer L the 3D matrix:

$$matrix[i] = matrix\_xyz, \text{ where } i = x + y*Lx + z*Lx*Ly \text{ (Eq. 1)}$$

as shown by block 940. The memory content allocated to the 3D matrix is written into an output file in binary format, as shown by block 950. The output file here is in a pseudo micro computed tomography (CT-like) image format, such that external applications configured to accept CT image files may accept the file as input.

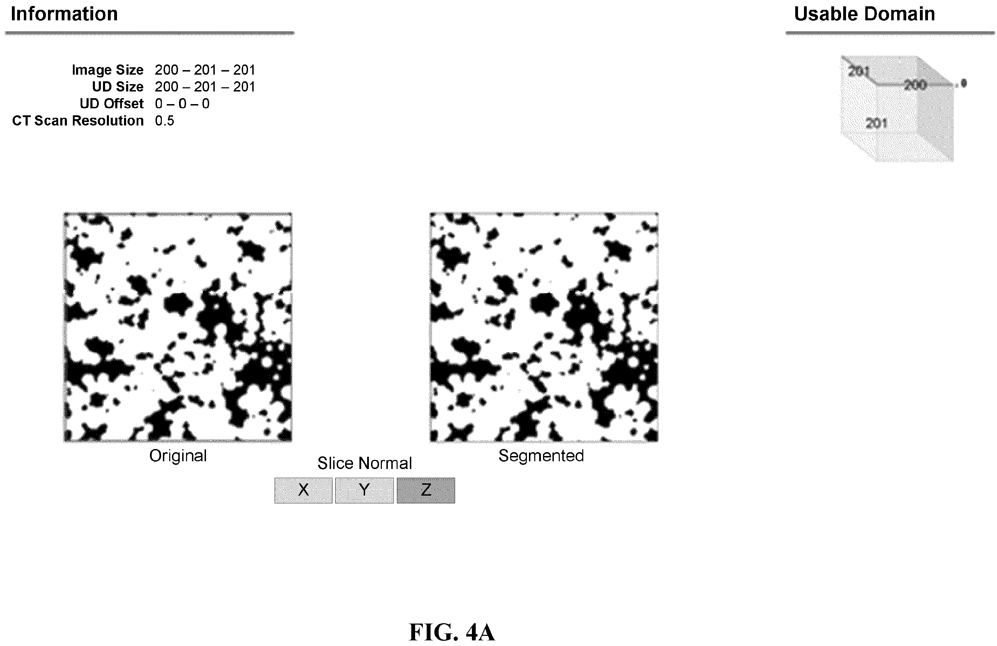

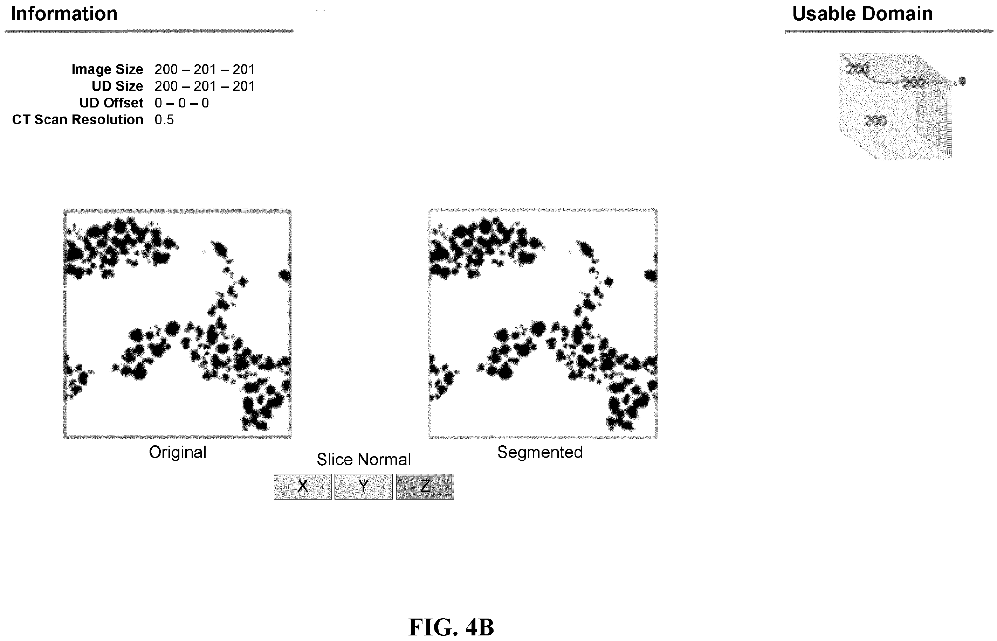

Under a fourth step of the first exemplary embodiment ("step 4"), the image file produced in the third step is uploaded into a material analysis application configured to accept CT images as input, for example DigitalROCK, where it is processed and converted to black and white images or grayscale images. FIG. 4A shows side-by-side original and processed images of the 3D structure from Materials Studio (Mesocite module) in DigitalROCK. FIG. 4B shows side-by-side original and processed images of the 3D structure from LAMMPS (DPP2 model) in DigitalROCK.

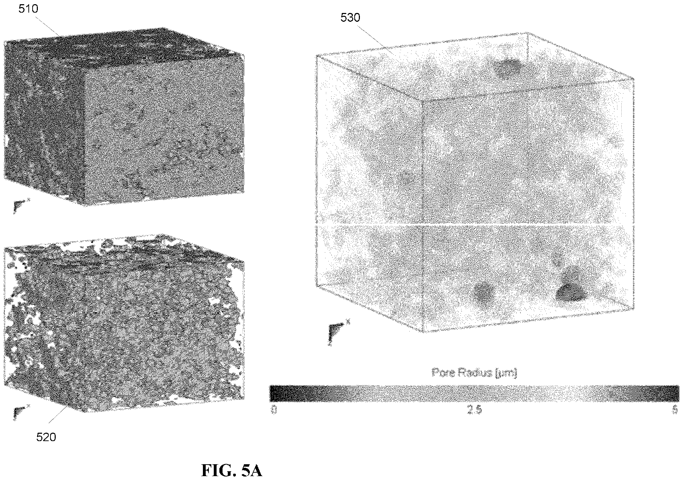

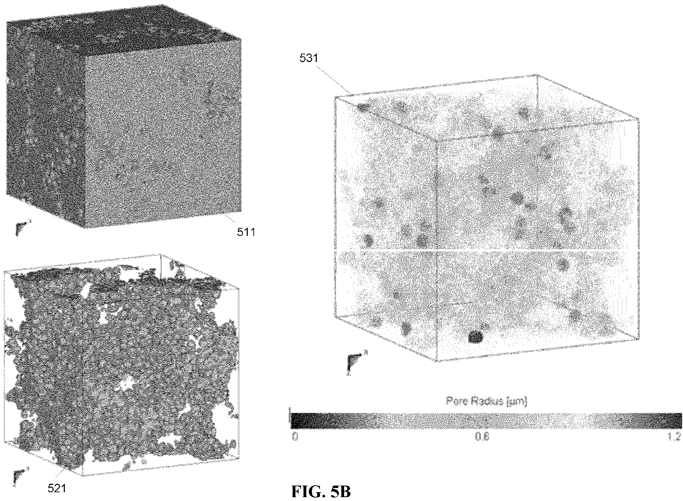

Under a fifth step of the first exemplary embodiment ("step 5"), after running PSA (pore size analysis) simulation in a material analysis application such as DigitalROCK, as shown by FIGS. 5A and 5B, the application may produce images for grains 510, 511 and pores 520, 521 of the material structure submitted via the CT-like image file.

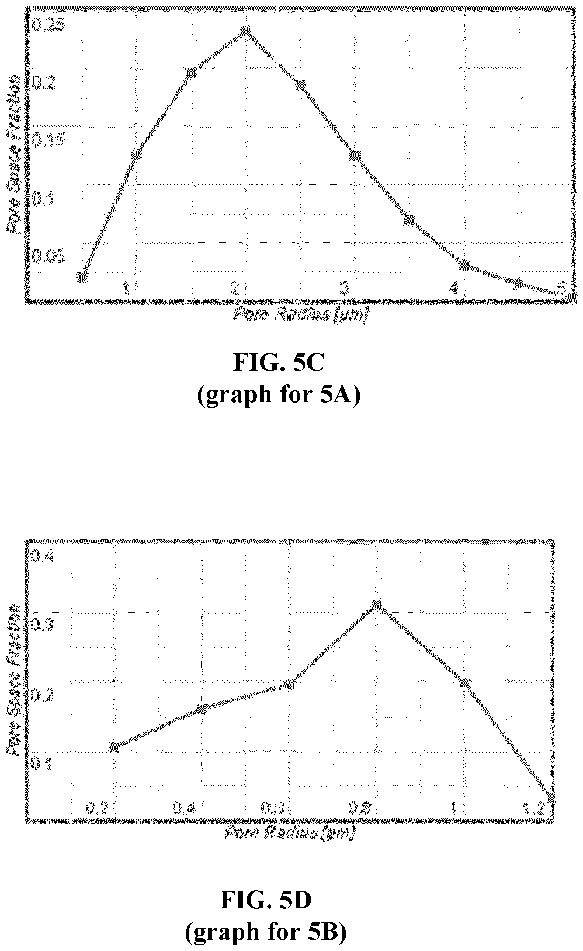

The analysis provided under the fifth step provides pore structures and distributions closer to those from experimental mercury porosimetry method, compared to the void volume analysis method that exploits the randomly placed probe material and determines if this probe material overlaps the particles in the system. While the performance of this conventional void volume analysis method depends on the type of the probe material and this method cannot examine void volumes, or pores, where the probe material cannot access, DigitalROCK can access whole pores based on the generated images. In addition, graphical pore size distribution 530, 531 inside the materials and the pore size distribution plot are also available as shown by FIGS. 5C and 5D.

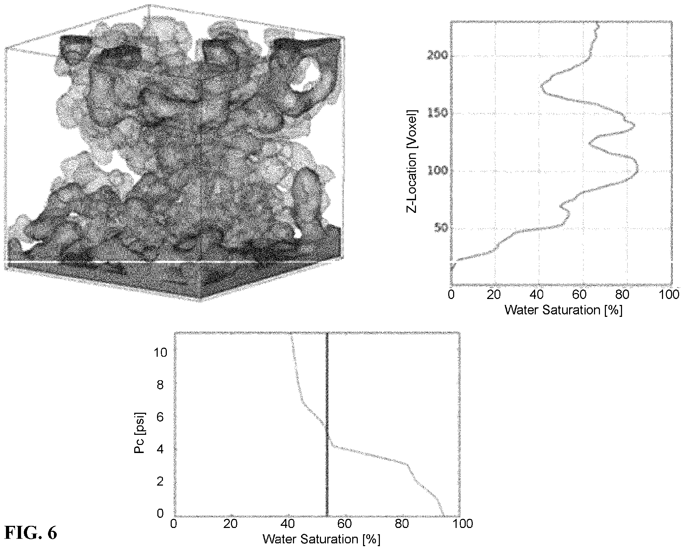

As shown by FIG. 6, fluid flow simulations may also be produced by the material analysis application for calculating properties like absolute and relative permeabilities, and capillary pressure as function of saturations of different types of fluids. Here, the electrolyte imbibition in the simulated porous electrode structures are shown, which visualizes the saturated pores inside the electrode. FIG. 6 shows a visualized pore structure of the material saturated by a liquid at a specific saturated concentration (top left), a plot of the liquid saturation concentration versus location in height, (top right) and the capillary pressure plot with respect to the liquid saturation (bottom).

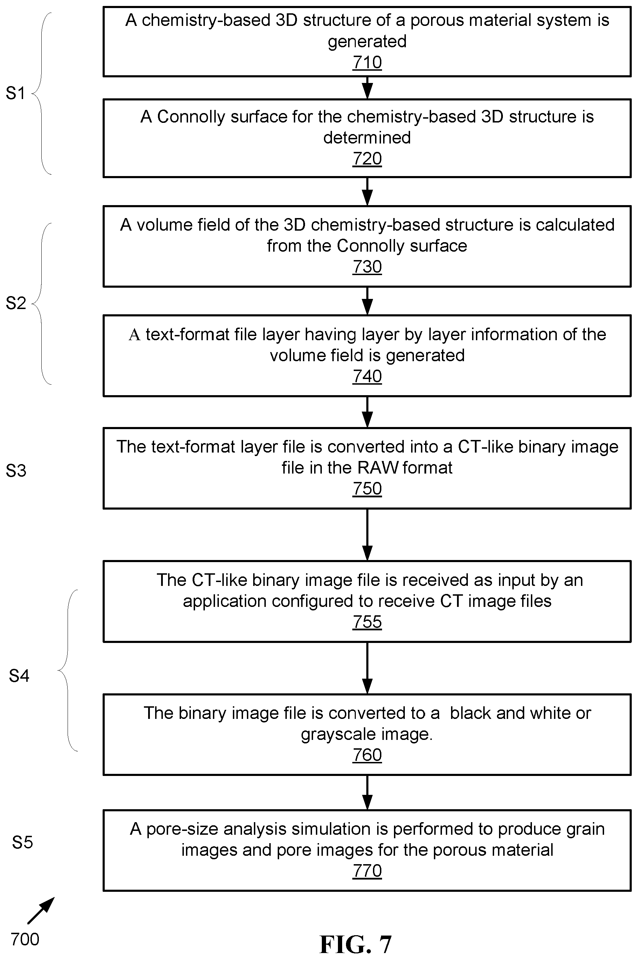

FIG. 7 is a flowchart summarizing the above described first exemplary method for generating a pseudo micro computed tomography (CT-like) image of a porous material. A chemistry-based 3D structure of a porous material system is built, as shown by block 710. For example, this may include calculating parameters for performing atomistic, coarse-grained, or DPD (dissipative particle dynamics) simulations, and running atomistic, coarse-grained, or DPD simulations. A Connolly surface for the chemistry-based 3D structure is determined, as shown by block 720. A volume field of the 3D chemistry-based structure from the Connolly surface is calculated, as shown by block 730. The volume field is sliced into layers (segments). A text-format file having layer by layer information of the volume field is generated (see FIG. 8), as shown by block 740. The sliced volume field is converted to RAW-format image files. First, the text-format layer file is converted into a binary image file in RAW format, as shown by block 750 (see FIG. 9). Next, the binary image file is converted to a CT-like black and white or grayscale image, as shown by block 760. A pore size analysis (PSA) simulation is performed to produce grain images and pore images for the porous material system, as shown by block 770. For example, the pore-scale simulation may model absolute/relative permeability and capillary pressure either via running scripts in a command-line interface or via the GUI (graphical user interface). In an exemplary implementation, a cloud based 3DEXPERIENCE platform ecosystem performs all the above steps and contains the 3D chemistry modeling, the data transfer, the pore-scale simulation of microstructural properties, and the optimization algorithms to search for optimal 3D chemistry models based on optimal microstructural properties.

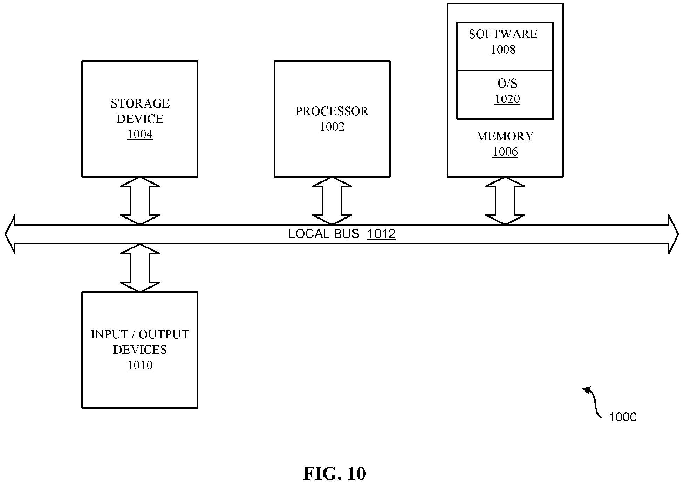

The present system for executing the functionality described in detail above may be a computer, an example of which is shown in the schematic diagram of FIG. 10. The system 1000 contains a processor 1002, a storage device 1004, a memory 1006 having software 1008 stored therein that defines the abovementioned functionality, input, and output (I/O) devices 1010 (or peripherals), and a local bus, or local interface 1012 allowing for communication within the system 1000. The local interface 1012 can be, for example but not limited to, one or more buses or other wired or wireless connections, as is known in the art. The local interface 1012 may have additional elements, which are omitted for simplicity, such as controllers, buffers (caches), drivers, repeaters, and receivers, to enable communications. Further, the local interface 1012 may include address, control, and/or data connections to enable appropriate communications among the aforementioned components.

The processor 1002 is a hardware device for executing software, particularly that stored in the memory 1006. The processor 1002 can be any custom made or commercially available single core or multi-core processor, a central processing unit (CPU), an auxiliary processor among several processors associated with the present system 1000, a semiconductor based microprocessor (in the form of a microchip or chip set), a macroprocessor, or generally any device for executing software instructions.

The memory 1006 can include any one or combination of volatile memory elements (e.g., random access memory (RAM, such as DRAM, SRAM, SDRAM, *etc.*)) and nonvolatile memory elements (*e.g.*, ROM, hard drive, tape, CDROM, *etc.*). Moreover, the memory 1006 may incorporate electronic, magnetic, optical, and/or other types of storage media. Note that the memory 1006 can have a distributed architecture, where various components are situated remotely from one another, but can be accessed by the processor 1002.

The software 1008 defines functionality performed by the system 1000, in accordance with the present invention. The software 1008 in the memory 1006 may include one or more separate programs, each of which contains an ordered listing of executable instructions for implementing logical functions of the system 1000, as described below. The memory 1006 may contain an operating system (O/S) 1020. The operating system essentially controls the execution of programs within the system 1000 and provides scheduling, input-output control, file and data management, memory management, and communication control and related services.

The I/O devices 1010 may include input devices, for example but not limited to, a keyboard, mouse, scanner, microphone, *etc.* Furthermore, the I/O devices 1010 may also include output devices, for example but not limited to, a printer, display, *etc.* Finally, the I/O devices 1010 may further include devices that communicate via both inputs and outputs, for instance but not limited to, a modulator/demodulator (modem; for accessing another device, system, or network), a radio frequency (RF) or other transceiver, a telephonic interface, a bridge, a router, or other device.

When the system 1000 is in operation, the processor 1002 is configured to execute the software 1008 stored within the memory 1006, to communicate data to and from the memory 1006, and to generally control operations of the system 1000 pursuant to the software 1008, as explained above.

When the functionality of the system 1000 is in operation, the processor 1002 is configured to execute the software 1008 stored within the memory 1006, to communicate data to and from the memory 1006, and to generally control operations of the system 1000 pursuant to the software 1008. The operating system 1020 is read by the processor 1002, perhaps buffered within the processor 1002, and then executed.

When the system 1000 is implemented in software 1008, it should be noted that instructions for implementing the system 1000 can be stored on any computer-readable medium for use by or in connection with any computer-related device, system, or method. Such a computer-readable medium may, in some embodiments, correspond to either or both the memory 1006 or the storage device 1004. In the context of this document, a computer-readable medium is an electronic, magnetic, optical, or other physical device or means that can contain or store a computer program for use by or in connection with a computer-related device, system, or method. Instructions for implementing the system can be embodied in any computer-readable medium for use by or in connection with the processor or other such instruction execution system, apparatus, or device. Although the processor 1002 has been mentioned by way of example, such instruction execution system, apparatus, or device may, in some embodiments, be any computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the processor or other such instruction execution system, apparatus, or device.

Such a computer-readable medium can be, for example but not limited to, an electronic, magnetic, optical, electromagnetic, infrared, or semiconductor system, apparatus, device, or propagation medium. More specific examples (a nonexhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program can be electronically captured, via for instance optical scanning of the paper or other medium, then compiled, interpreted, or otherwise processed in a suitable manner if necessary, and then stored in a computer memory.

In an alternative embodiment, where the system 1000 is implemented in hardware, the system 1000 can be implemented with any or a combination of the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), etc.

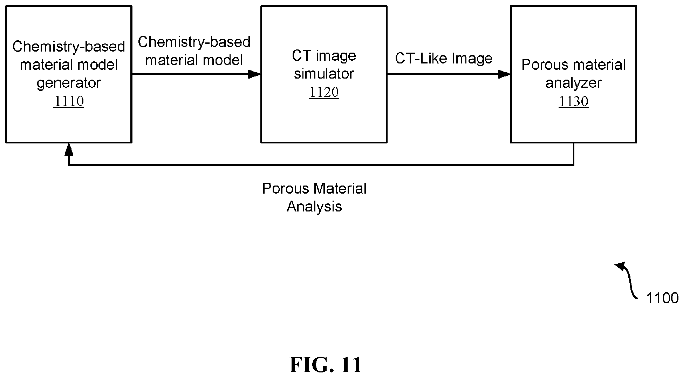

FIG. 11 is a schematic diagram of an exemplary embodiment of a system 1110 for modeling a porous material, generating a pseudo micro computed tomography (CT-like) image of the porous material, and analyzing the porous material for manufacture. Conceptually, the computer based system may be broadly divided into the three modules 1110, 1120, 1130. A chemistry-based material model generator 1110 is configured to generate a chemistry-based 3D model of the porous material, for example, by generating a mesoscale 3D structure and/or a coarse-grained DPP2 model as described above. A Connolly surface for the chemistry-based 3D structure is determined.

A CT image simulator 1120 calculates a volume field of the 3D chemistry-based structure from the Connolly surface, generates a text-format file having layer by layer information of the volume field, and converts the text-format layer file into a CT-like binary image file in RAW format, as described above. A porous material analyzer 1130 receiving the CT-like image file, converts the image file to a 3D image of the porous material, and performs a pore size analysis (PSA) to produce grain images and pore images for the porous material system, as described above. For example, the porous material analyzer may be the DigitalROCK platform, and may be used to simulate the porous material under various conditions. The results of the porous material analyzer may be used to alter the chemistry-based material model in subsequent process iterations as the desired characteristics of the porous material are developed. Thereafter, the porous material analyzer 1130 may be used to control a manufacturing process to produce the porous material.

The modules 1110, 1120, 1130 may be separate software applications running on separate processors. Alternatively, two or more of the modules 1110, 1120, 1130 may run on the same processors. Further, the functionality of two or more of the modules 1110, 1120, 1130 may co-exist in the same software package or environment. For example, the CT image simulator 1120 may be implemented as a script within the chemistry-based material model generator 1110. Further one or more of the modules 1110, 1120, 1130 may include a number of sub-processes, and/or may interact with external applications to perform their functions.

Advantageously, the exemplary embodiments do not require any experimental inputs such as real micro-CT or FIB-SEM images of the porous materials. Starting from the chemistry of the material, the 3D microscopic structure can be simulated from which the input image files can be generated. The workflow presented in the above described embodiments represents a more ergonomic, faster, more efficient, and less expensive approach from the previous approach that involved acquiring the materials, processing the materials, and then imaging the materials for analysis, including but not limited to pore analysis, calculation of capillary pressure and permeability, and fluid flow simulation even without synthesizing real (non-simulated) materials.

The embodiments present a first-of-a-kind workflow for this purpose. Potential applications can involve modeling of flow behavior in human membrane or tissue such as drug transport, blood flow, skin permeability, or other flow- and transport-related behavior for medical and pharmaceutical applications. Additional applications include the design of orthopedic scaffolds, where porosity, pore size, pore shape and pore distribution are crucial factors in bone regeneration in the field of bone/tissue engineering.

The embodiments may increase sustainability by reducing consumption and waste of materials through virtual testing and analysis prior to manufacturing, where a global optimization search for best microstructures can be done automatically in modeling and simulation ecosystems such as the 3DExperience platform. In addition, animal tests and risky clinical trials on patients may be reduced or even replaced. Moreover, design or improvement of filter systems based on porous materials or porous energy storage materials are potential applications of this invention as it allows to reduce energy and material consumption. In addition to application in healthcare and personal care areas, this invention can also be applicable to battery cells, fuel cells and other energy devices. In the area of sustainability, the embodiments may be of tremendous help in evaluating the economics of fluid injection (including $CO_2$ storage) and migration in geochemical applications.

The embodiments of the invention produce a CT-like image file of a material that cannot possibly or practically be scanned by known scanning processes. The CT-like image file may then be received and processed by subsequent applications in the same manner as a CT image produced by a CT scanner.

The technical effect of this invention is to produce a CT-like image of a material in scenarios where producing an actual scan of the material is impractical or impossible. The CT-like image produced by the embodiments may be used in lieu of an actual CT image, for example, when the CT-like image is received as input by an application that is configured to receive an actual CT image. For example, the CT-like image may be used during the manufacturing of the material that (previously) was not scannable. For example, a subsequent simulation based on the CT-like image may be used to define the manufacturing process of a corresponding real-world material. For example, the simulation may determine ranges of material properties for a component of the manufactured material.

In general, the embodiments produce a necessary input (the CT-like image) for subsequent simulation of the material (e.g., in DigitalROCK), where the subsequent simulation results in alteration the design of a real-world material, for example, to improve the physical characteristics of the real-world material, such as improved energy conversion efficiency, improved fluid flow characteristics, improved heat dissipation, improved operational characteristics of the real-world material, and/or to improve the manufacturing process of the real-world material (for example, lower energy consumption for the manufacturing process, higher yield rates of the manufacturing process, faster production efficiency for the manufacturing process).

While the embodiments described above generally refer to spherical shaped particles, however, a person having ordinary skill in the art would be able to apply the above teachings to particles having other (non-spherical) shapes.

**Claims**

1. A computer-based method for generating a pseudo micro computed tomography (CT-like) image of a porous material for manufacture comprising the steps of:

   generating a chemistry-based three-dimensional (3D) structure of the porous material (710);
   determining a Connolly surface for the chemistry-based 3D structure (720);
   calculating a volume field of the 3D chemistry-based structure from the Connolly surface (730);
   generating a text-format file having layer by layer information of the volume field (740); and
   converting the text-format layer file into a CT-like binary image file in RAW format (750).

2. The method of claim 1, further comprising the steps of:

   converting the binary image file to an image; and
   performing a pore size analysis (PSA) to produce grain images and pore images for the porous material system.

3. The method of claim 1, wherein generating the text-format file having layer by layer information of the volume field further comprises the steps of:

   plotting a 3D grid inside the 3D chemistry-based structure with pre-defined increments in x, y, and z axes;
   for each point of the 3D grid, determining whether the position of the corresponding volume field corresponds to a particle or to a pore; and
   based on the 3D grid, writing a resulting xyz matrix to a text format layer file in a binary format representing each grid position as either a particle or a pore.

4. The method of claim 3, wherein converting the text-format layer file into the binary image file in RAW format further comprises the steps of:

   opening the text-format layer file;
   allocating a memory space for a 3D matrix;
   reading sequentially the values for x, y, z, of the xyz matrix from the text-format layer file;
   assigning numerical values to the elements of the 3D matrix; and
   writing the memory content allocated to the 3D matrix into an output file in binary format.

5. The method of claim 1, wherein generating a chemistry-based 3D structure of a porous material system further comprises the steps of modeling the porous material at a mesoscale level.

6. The method of claim 5, wherein modeling the porous material at a mesoscale level further comprises dissipative particle dynamics (DPD) simulation.

7. The method of claim 6, further comprising the step of generating DPD forcefield input parameters using the solubility parameter of each particle in the porous material system.

8. The method of claim 7, further comprising the step of

   determining energy minimization and equilibration of the porous material system; and
   running a DPD simulation to obtain mechanical properties of the porous material.

9. The method of claim 8, wherein determining mechanical properties of the porous material comprises using experimental mechanical properties of a similar material.

10. The method of claim 8, further comprising the step of if experimental data are not available, obtaining the mechanical properties of the porous material from all-atom molecular dynamics (MD) simulations.

11. The method of claim 5, wherein modeling the porous material at a mesoscale level further comprises coarse-grained (CG) simulation.

12. The method of claim 11, further comprising the steps of:

   constructing a coarse-grained initial structure by placing particles randomly inside an elementary volume; and running a CG molecular dynamics (MD) simulation in isothermal isobaric condition after energy minimization to adjust a density of the material.

13. A computer program comprising instructions, which, when the program is executed by a computer, cause the computer to perform the method according to any one of claims 1 to 12.

14. A computer readable medium having recorded thereon the computer program of claim 13.

15. A system for modeling a porous material, generating a pseudo micro computed tomography (CT-like) image of the porous material, and analyzing the porous material for manufacture, comprising:
a processor and a memory configured to store non-transitory instructions that, when executed by the processors, implements the following application modules:

   a chemistry-based material model generator configured to perform the steps of:

      generating a chemistry-based three-dimensional (3D) structure of the porous material; and
      determining a Connolly surface for the chemistry-based 3D structure;

   a CT image simulator configured to perform the steps of:

      calculating a volume field of the 3D chemistry-based structure from the Connolly surface;
      generating a text-format file having layer by layer information of the volume field; and
      converting the text-format layer file into a CT-like binary image file in RAW format; and

   a porous material analyzer, configured to perform the steps of:

      receiving the binary image file;
      converting the binary image file to a 3D image of the porous material; and performing a pore size analysis (PSA) to produce grain images and pore images for the porous material system.


**Patentansprüche**

1. Computergestütztes Verfahren zum Erzeugen eines pseudo-mikrocomputertomographischen (CT-ähnlichen) Bilds eines porösen Materials zur Herstellung, umfassend die folgenden Schritte:

   Erzeugen einer chemiebasierten dreidimensionalen (3D) Struktur des porösen Materials (710);
   Bestimmen einer Connolly-Oberfläche für die chemiebasierte 3D-Struktur (720);
   Berechnen eines Volumenfelds der chemiebasierten 3D-Struktur aus der Connolly-Oberfläche (730);
   Erzeugen einer Textformatdatei, die schichtweise Informationen des Volumenfelds (740) aufweist; und
   Konvertieren der Textformatschichtdatei in eine CT-ähnliche binäre Bilddatei im RAW-Format (750).

2. Verfahren nach Anspruch 1, ferner umfassend die folgenden Schritte:

   Konvertieren der binären Bilddatei in ein Bild; und
   Durchführen einer Porengrößenanalyse (PSA) zum Erzeugen von Korn- und Porenbildern für das poröse Materialsystem.

3. Verfahren nach Anspruch 1, wobei das Erzeugen der Textformatdatei, die schichtweise Informationen des Volumenfelds aufweist, ferner die folgenden Schritte umfasst:

   Zeichnen eines 3D-Gitters innerhalb der chemiebasierten 3D-Struktur mit vordefinierten Inkrementen in den Achsen x, y und z;

für jeden Punkt des 3D-Gitters, Bestimmen, ob die Position des entsprechenden Volumenfelds einem Partikel oder einer Pore entspricht; und

Basierend auf dem 3D-Gitters, Schreiben einer resultierenden xyz-Matrix in eine Textformatschichtdatei in einem binären Format, das jede Gitterposition entweder als Partikel oder als Pore darstellt.

4. Verfahren nach Anspruch 3, wobei ein Konvertieren der Textformatschichtdatei in die binäre Bilddatei im RAW-Format ferner die folgenden Schritte umfasst:

Öffnen der Textformatschichtdatei;

Zuweisen eines Speicherplatzes für eine 3D-Matrix;

sequentielles Lesen der Werte für x, y, z der xyz-Matrix aus der Textformatschichtdatei;

Zuweisen numerischer Werte zu den Elementen der 3D-Matrix; und

Schreiben des Speicherinhalts, der der 3D-Matrix zugeordnet ist, in eine Ausgabedatei in Binärformat.

5. Verfahren nach Anspruch 1, wobei ein Erzeugen einer chemiebasierten 3D-Struktur eines porösen Materialsystems ferner die Schritte eines Modellierens des porösen Materials auf einer Mesoskalenebene umfasst.

6. Verfahren nach Anspruch 5, wobei ein Modellieren des porösen Materials auf Mesoskalenebene ferner eine Simulation dissipativer Teilchendynamik (DPD) umfasst.

7. Verfahren nach Anspruch 6, ferner umfassend den Schritt eines Erzeugens von DPD-Kraftfeldeingabeparametern unter Verwendung des Löslichkeitsparameters von jedem Teilchen in dem porösen Materialsystem.

8. Verfahren nach Anspruch 7, ferner umfassend den folgenden Schritt

Bestimmen von Energieminimierung und Gleichgewicht des porösen Materialsystems; und

Durchführen einer DPD-Simulation, um mechanische Eigenschaften des porösen Materials zu erlangen.

9. Verfahren nach Anspruch 8, wobei ein Bestimmen mechanischer Eigenschaften des porösen Materials ein Verwenden experimenteller mechanischer Eigenschaften eines ähnlichen Materials umfasst.

10. Verfahren nach Anspruch 8, ferner umfassend den Schritt, wenn keine experimentellen Daten verfügbar sind, eines Erlangens der mechanischen Eigenschaften des porösen Materials aus Simulationen der molekularen Dynamik (MD) auf der Basis aller Atome.

11. Verfahren nach Anspruch 5, wobei ein Modellieren des porösen Materials auf einer Mesoskalenebene ferner eine grobkörnige (CG) Simulation umfasst.

12. Verfahren nach Anspruch 11, ferner umfassend die folgenden Schritte:

Aufbauen einer grobkörnigen Ausgangsstruktur durch zufälliges Platzieren von Teilchen innerhalb eines Elementarvolumens; und

Durchführung einer Simulation von CG-Molekulardynamik (MD) unter isothermen, isobaren Bedingungen nach Energieminimierung, um eine Dichte des Materials anzupassen.

13. Computerprogramm, umfassend Anweisungen, die, wenn das Programm von einem Computer ausgeführt wird, den Computer veranlassen, das Verfahren nach einem der Ansprüche 1 bis 12 durchzuführen.

14. Computerlesbares Medium, auf dem das Computerprogramm nach Anspruch 13 aufgezeichnet ist.

15. System zum Modellieren eines porösen Materials, zum Erzeugen eines pseudo-mikrocomputertomographischen (CT-ähnlichen) Bilds des porösen Materials und zum Analysieren des porösen Materials zur Herstellung, umfassend: einen Prozessor und einen Speicher, der konfiguriert ist, um nichttransitorische Anweisungen zu speichern, die, wenn sie von den Prozessoren ausgeführt werden, die folgenden Anwendungsmodule implementieren: einen chemiebasierten Materialmodellgenerator, der konfiguriert ist, um die folgenden Schritte durchführen:

Erzeugen einer chemiebasierten dreidimensionalen (3D) Struktur des porösen Materials; und

Bestimmen einer Connolly-Oberfläche für die chemiebasierte 3D-Struktur;
einen CT-Bildsimulator, der konfiguriert ist, um die folgenden Schritte durchzuführen:

Berechnen eines Volumenfelds der chemiebasierten 3D-Struktur aus der Connolly-Oberfläche;
Erzeugen einer Textformatdatei, die schichtweise Informationen des Volumenfelds aufweist; und
Konvertieren der Textformatschichtdatei in eine CT-ähnliche binäre Bilddatei im RAW-Format; und
einen Analysator für poröses Material, der konfiguriert ist, um die folgenden Schritte durchzuführen:

Empfangen der binären Bilddatei;
Konvertieren der binären Bilddatei in ein 3D-Bild des porösen Materials; und
Durchführen einer Porengrößenanalyse (PSA), um Korn- und Porenbilder für das poröse Materialsystem zu erzeugen.

**Revendications**

1. Procédé mis en œuvre par ordinateur pour générer une image de pseudo-microtomographie (de type CT) d'un matériau poreux en vue de sa fabrication, comprenant les étapes comprenant le fait de :

générer une structure tridimensionnelle (3D) basée sur la chimie du matériau poreux (710) ;
déterminer une surface de Connolly pour la structure 3D basée sur la chimie (720) ;
calculer un champ de volume de la structure 3D basée sur la chimie à partir de la surface de Connolly (730) ;
générer un fichier au format texte contenant des informations couche par couche du champ de volume (740) ; et
convertir le fichier de couche au format texte en un fichier image binaire de type CT au format RAW (750).

2. Procédé selon la revendication 1, comprenant en outre les étapes comprenant le fait de :

convertir le fichier image binaire en une image ; et
effectuer une analyse de la taille des pores (PSA) afin de produire des images de grains et des images de pores pour le système de matériaux poreux.

3. Procédé selon la revendication 1, dans lequel la génération du fichier au format texte contenant des informations couche par couche du champ de volume comprend en outre les étapes comprenant le fait de :

tracer une grille 3D à l'intérieur de la structure 3D basée sur la chimie avec des incréments prédéfinis sur les axes x, y et z ;
pour chaque point de la grille 3D, déterminer si la position du champ de volume correspondant correspond à une particule ou à un pore ; et
sur la base de la grille 3D, écrire une matrice xyz résultante dans un fichier de couche au format texte dans un format binaire représentant chaque position de grille comme étant soit une particule soit un pore.

4. Procédé selon la revendication 3, dans lequel la conversion du fichier de couche au format texte en le fichier image binaire au format RAW comprend en outre les étapes comprenant le fait de :

ouvrir le fichier de couche au format texte ;
allouer un espace mémoire pour une matrice 3D ;
lire séquentiellement les valeurs pour x, y, z de la matrice xyz à partir du fichier de couche au format texte ;
attribuer des valeurs numériques aux éléments de la matrice 3D ; et
écrire le contenu de mémoire alloué à la matrice 3D dans un fichier de sortie au format binaire.

5. Procédé selon la revendication 1, dans lequel la génération d'une structure 3D basée sur la chimie d'un système de matériaux poreux comprend en outre l'étape de modélisation du matériau poreux à une échelle mésoscopique.

6. Procédé selon la revendication 5, dans lequel la modélisation du matériau poreux à une échelle mésoscopique comprend en outre une simulation de dynamique des particules dissipatives (DPD).

7. Procédé selon la revendication 6, comprenant en outre l'étape de génération de paramètres d'entrée de champ de force de dynamique DPD en utilisant le paramètre de solubilité de chaque particule dans le système de matériaux poreux.

8. Procédé selon la revendication 7, comprenant en outre l'étape comprenant le fait de :

déterminer un équilibrage et une minimisation d'énergie du système de matériaux poreux ; et
exécuter une simulation DPD afin d'obtenir les propriétés mécaniques du matériau poreux.

9. Procédé selon la revendication 8, dans lequel la détermination des propriétés mécaniques du matériau poreux comprend l'utilisation des propriétés mécaniques expérimentales d'un matériau similaire.

10. Procédé selon la revendication 8, comprenant en outre l'étape comprenant le fait de, si des données expérimentales ne sont pas disponibles, obtenir les propriétés mécaniques du matériau poreux à partir de simulations de dynamique moléculaire (MD) tous atomes.

11. Procédé selon la revendication 5, dans lequel la modélisation du matériau poreux à une échelle mésoscopique comprend en outre une simulation à gros grains (CG).

12. Procédé selon la revendication 11, comprenant en outre les étapes comprenant le fait de :

construire une structure initiale à gros grains en plaçant des particules de manière aléatoire à l'intérieur d'un volume élémentaire ; et
exécuter une simulation de dynamique moléculaire (MD) à gros grains (CG) dans des conditions isothermes et isobares après la minimisation d'énergie afin d'ajuster la densité du matériau.

13. Programme informatique comprenant des instructions qui, lorsque le programme est exécuté par un ordinateur, amènent l'ordinateur à mettre en œuvre le procédé selon l'une quelconque des revendications 1 à 12.

14. Support lisible par ordinateur sur lequel est enregistré le programme informatique selon la revendication 13.

15. Système destiné à modéliser un matériau poreux, à générer une image de pseudo-microtomographie (de type CT) du matériau poreux et à analyser le matériau poreux en vue de sa fabrication, comprenant :
un processeur, et une mémoire configurée pour stocker des instructions non transitoires qui, lorsqu'elles sont exécutées par le processeur, mettent en œuvre les modules d'application suivants :

un générateur de modèle de matériau basé sur la chimie configuré pour effectuer les étapes comprenant le fait de :

générer une structure tridimensionnelle (3D) basée sur la chimie du matériau poreux ; et
déterminer une surface de Connolly pour la structure 3D basée sur la chimie ;

un simulateur d'image CT configuré pour effectuer les étapes comprenant le fait de :

calculer un champ de volume de la structure 3D basée sur la chimie à partir de la surface de Connolly ;
générer un fichier au format texte contenant des informations couche par couche du champ de volume ; et
convertir le fichier de couche au format texte en un fichier image binaire de type CT au format RAW ; et

un analyseur de matériaux poreux, configuré pour effectuer les étapes comprenant le fait de :

recevoir le fichier image binaire ;
convertir le fichier image binaire en une image 3D du matériau poreux ; et
effectuer une analyse de la taille des pores (PSA) afin de produire des images de grains et des images de pores pour le système de matériaux poreux.

**FIG. 1A**

**FIG. 1B**

FIG. 2A

FIG. 2B

FIG. 3A

FIG. 3B

**Information**

Image Size 200 – 201 – 201
UD Size 200 – 201 – 201
UD Offset 0 – 0 – 0
CT Scan Resolution 0.5

**Usable Domain**

Original

Slice Normal

| X | Y | Z |

Segmented

FIG. 4A

EP 4 459 494 B1

## Information

Image Size 200 – 201 – 201
UD Size 200 – 201 – 201
UD Offset 0 – 0 – 0
CT Scan Resolution 0.5

## Usable Domain

Original

Segmented

Slice Normal

| X | Y | Z |

**FIG. 4B**

EP 4 459 494 B1

**FIG. 5A**

FIG. 5B

**FIG. 5C**
**(graph for 5A)**

**FIG. 5D**
**(graph for 5B)**

FIG. 6

S1 {

A chemistry-based 3D structure of a porous material system is generated
710

A Connolly surface for the chemistry-based 3D structure is determined
720

S2 {

A volume field of the 3D chemistry-based structure is calculated from the Connolly surface
730

A text-format file layer having layer by layer information of the volume field is generated
740

S3

The text-format layer file is converted into a CT-like binary image file in the RAW format
750

S4 {

The CT-like binary image file is received as input by an application configured to receive CT image files
755

The binary image file is converted to a black and white or grayscale image.
760

S5

A pore-size analysis simulation is performed to produce grain images and pore images for the porous material
770

700

**FIG. 7**

Generate a 3D grid inside the target molecular structure with pre-defined increments in x, y, and z axes.
810

For each point of the 3D grid, determine whether the position of the corresponding volume field is occupied (particle) or is vacant (pore).
820

Write the resulting xyz matrix to a text layer file representing each grid point as a particle or a pore.
830

740

**FIG. 8**

Open the text-format layer file
910

Allocate a memory space for a 3D matrix of dimensions Lx, Ly, Lz, and data type float
920

Read sequentially the values for x, y, z, of the xyz matrix from the text-format layer file
930

Assign numerical values to the elements of the 3D matrix
940

Write the memory content allocated to the 3D matrix into an output file in binary format.
950

750

**FIG. 9**

STORAGE
DEVICE
1004

PROCESSOR
1002

SOFTWARE
1008

O/S
1020

MEMORY
1006

LOCAL BUS 1012

INPUT / OUTPUT
DEVICES
1010

1000

**FIG. 10**

Chemistry-based material model generator **1110** — Chemistry-based material model → CT image simulator **1120** — CT-Like Image → Porous material analyzer **1130**

Porous Material Analysis

1100

**FIG. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TIAGO SIMÕES et al.** *Geometric Detection Algorithms for Cavities on Protein Surfaces in Molecular Graphics: A Survey*